# EUROPEAN PATENT SPECIFICATION

(11) **EP 3 774 961 B1**
(45) Date of publication and mention of the grant of the patent: **15.01.2025**
(21) Application number: 19717880.9
(22) Date of filing: 12.04.2019
(51) Int. Cl.: C08G 18/02, C08G 18/20, C07C 269/02, C08G 18/72, C08G 18/28, C08G 18/18, C08G 18/76, C08G 18/73, C08G 18/71, C08J 5/04, C08G 18/79, C07C 273/18

(54) **ADDUCTS OF AMINE CATALYSTS FOR PRODUCING ISOCYANURATE POLYMERS**
ADDUKTE VON AMINKATALYSATOREN ZUR HERSTELLUNG VON ISOCYANURAT-POLYMEREN
PRODUITS D'ADDITION DE CATALYSEURS D'AMINE DESTINÉS À LA PRODUCTION DE POLYMÈRES À MOTIFS ISOCYANURATES

(30) Priority: 13.04.2018 EP 18167245
(43) Date of publication of application: 17.02.2021
(73) Proprietor: Covestro Deutschland AG, 51373 Leverkusen (DE)
(72) Inventor: MEISENHEIMER, Richard, Daniel, Matthias, 51065 Köln (DE); HEINZ, Paul, 51375 Leverkusen (DE); ACHTEN, Dirk, 51375 Leverkusen (DE); STERN, Frank-Stefan, 51427 Bergisch Gladbach (DE); HECKING, Andreas, 40764 Langenfeld (DE); STROBEL, Fee, 50823 Köln (DE)
(74) Representative: Levpat
(86) International application number: PCT/EP2019/059481
(87) International publication number: WO 2019/197638

(56) References cited:
- WO-A1-2018/054776
- WO-A1-2019/096813
- US-A- 4 026 840
- US-A- 4 122 038
- US-A1- 2008 090 943

## Description

The present invention relates to urethane, thiourethane and urea adducts of tertiary amines and the use thereof as catalysts for the crosslinking of aliphatically, cycloaliphatically, araliphatically or aromatically bonded isocyanate groups with one another. The catalysts according to the invention have the particular advantage that they are thermolatent. The invention is set out in the appended set of claims.

The production of isocyanurate plastics by the crosslinking of aliphatically or cycloaliphatically bonded isocyanate groups with one another, i.e. without the involvement of thiol, hydroxyl or amino groups, is known per se. It has also been described previously that such materials are employable as a polymer matrix for composite materials.

Most of the catalysts for the trimerization reaction of aromatic isocyanates to afford PIR (rigid polyisocyanurate foams) which is well known in the prior art are not suitable for catalysis of the trimerization reaction of the much less reactive aliphatic isocyanates. The catalysts described in the literature are either insufficiently reactive and require long curing times at high temperatures or excessively reactive and do not allow controlled progress of the reaction.

While aromatic isocyanates are to a large extent converted into rigid foams and flexible foams where a rapid onset of reaction is desired, aliphatic isocyanates are mainly employed in paints and adhesives and also in potting compounds as crosslinking components for producing polyurethanes. In these applications catalysts allowing a long pot life of the reaction mixture and a short reaction time at elevated temperature are preferred. In the case of aromatic isocyanates long pot lives are achievable only through the use of additional inhibitors on account of their high reactivity.

The need for a catalyst having a long pot life at room temperature and a high rate after heating exists in particular in the production of composite materials comprising a polymer matrix of trimerized aliphatic isocyanates by pultrusion. This process is often used for continuous and thus particularly economic production of composite materials.

The production of fibre-composite materials based on aliphatic or cycloaliphatic polyisocyanates having a high ratio of isocyanate groups to hydroxyl, thiol and amino groups in the reaction mixture by pultrusion has not yet been described in public. Such a process requires a catalyst which at room temperature shows only low catalytic acitivity - ideally none whatsoever - and after temperature elevation brings about a very rapid crosslinking of the isocyanate groups in the reaction mixture. The low activity at room temperature allows long-term storage of the reaction mixture of polyisocyanate and catalyst without the incipient crosslinking of the polyisocyanates resulting in a viscosity increase which impedes or renders impossible further processing. Also desirable is a high catalytic activity after activation of the catalyst to achieve high production speeds. Since the activation of the catalysts used in the conventional systems is effected by heating, a catalyst which crosslinks isocyanate groups should likewise be activatable by heating. This would provide a system composed of catalyst and (cyclo)aliphatic polyisocyanate which is processable in the machines conventionally employed for pultrusion without requiring the machines employed to be adapted greatly.

The production of composite materials with polymer matrices constructed from unsaturated polyester resin (UP resin) or epoxy resins is already known. The pultrusion process is also known for producing these materials. The disadvantage of UP resin is the severe odour development resulting from the styrene present. In addition, the mechanical properties such as for example tensile elastic modulus or flexural strength are generally not sufficiently well developed. UP resin also has very low adhesive properties. The disadvantage of epoxy resins is that the ratio of epoxy resin to hardener must be very precisely observed since deviations have a severe impact on material properties. Neither material is permanently weather-resistant.

A pultrusion process based on the crosslinking of aliphatic polyisocyanates was described for the first time in patent application WO 2018/054776 A. This document also discloses a suitable catalyst system. However, the use of polyethylene glycol described therein results in poorer mechanical properties of the polymer matrix. Systems which have an even longer pot life but can nevertheless be cured at lower temperatures than the known system are also desirable for applications other than pultrusion. The initial viscosity of the resin should also be as low as possible in order to facilitate processing.

This problem is solved by the embodiments of the present invention that are disclosed in the claims and in this description.

In a first embodiment the present invention relates to an adduct of a compound of formula (I) and a compound having at least one isocyanate group 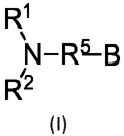
wherein R¹ and R² are independently of one another selected from the group consisting of hydrogen, methyl, ethyl, propyl, isopropyl, butyl and isobutyl, branched C5-alkyl, unbranched C5-alkyl, branched C6-alkyl, unbranched C6-alkyl, branched C7-alkyl and unbranched C7-alkyl;
R⁵ is selected from the group consisting of propylene, butylene, pentylene and a radical of formula (II), preferably from butylene and the radical of formula (II); 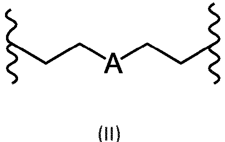
   wherein A in formula (II) is selected from the group consisting of O, S and NR³,
   wherein R³ is selected from the group consisting of H, methyl, ethyl, propyl, isopropyl, butyl und isobutyl, preferably H and methyl; and
B is independently of A selected from the group consisting of OH, SH NHR⁴ and NH₂, wherein
R⁴ is selected from the group consisting of methyl, ethyl and propyl, preferably methyl.

### Preferred variants of the compound of formula (I)

In a preferred embodiment of the present invention R⁵ is a radical of formula (II), wherein A is NR³ and R³ is selected from the group consisting of hydrogen, methyl, ethyl, propyl, isopropyl, butyl and isobutyl. It is preferable when R³ is H, methyl or ethyl. It is particularly preferable when R³ is methyl.
In a first variant of this embodiment B is OH and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.
In a second variant of this embodiment B is SH and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.
In a third variant of this embodiment B is NHR⁴ and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl. In this variant R⁴ is selected from the group consisting of methyl, ethyl and propyl. It is preferable when R⁴ is H, methyl or ethyl. It is particularly preferable when R⁴ is methyl.
In a fourth variant of this embodiment B is NH₂ and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.

In a further preferred embodiment of this invention R⁵ is a radical according to formula (II), wherein A is oxygen.
In a first variant of this embodiment B is OH and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.
In a second variant of this embodiment B is SH and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.
In a third variant of this embodiment B is NHR⁴ and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another methyl or ethyl. It is particularly preferable when R¹ and R² are methyl. In this variant R⁴ is selected from the group consisting of methyl, ethyl and propyl. It is preferable when R⁴ is H, methyl or ethyl. It is particularly preferable when R⁴ is methyl.
In a fourth variant of this embodiment B is NH₂ and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.

In yet a further preferred embodiment of this invention R⁵ is a radical according to formula (II), wherein A is sulfur.
In a first variant of this embodiment B is OH and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.
In a second variant of this embodiment B is SH and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.
In a third variant of this embodiment B is NHR⁴ and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another methyl or ethyl. It is particularly preferable when R¹ and R² are methyl. In this variant R⁴ is selected from the group consisting of methyl, ethyl and propyl. It is preferable when R⁴ is H, methyl or ethyl. It is particularly preferable when R⁴ is methyl.
In a fourth variant of this embodiment B is NH₂ and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.

In yet a further preferred embodiment of this invention R⁵ is a butylene radical.
In a first variant of this embodiment B is OH and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.
In a second variant of this embodiment B is SH and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.
In a third variant of this embodiment B is NHR⁴ and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another methyl or ethyl. It is particularly preferable when R¹ and R² are methyl. In this variant R⁴ is selected from the group consisting of methyl, ethyl and propyl. It is preferable when R⁴ is H, methyl or ethyl. It is particularly preferable when R⁴ is methyl.
In a fourth variant of this embodiment B is NH₂ and R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl. It is preferable when R¹ and R² are independently of one another H, methyl or ethyl. It is particularly preferable when R¹ and R² are methyl.

In the context of the study underlying the present invention it has surprisingly been found that it is advantageous when the abovementioned compounds are not added to a crosslinkable polyisocyanate as such. The use of one of the below-defined adducts of one of the above-defined compounds of formula (I) has the result that a reaction mixture of polyisocyanate and catalyst (i) exhibits a lower viscosity from the outset, (ii) that the viscosity of this reaction mixture at temperatures below 50°C increases more slowly than in the case of the system known from PCT/EP2017/073276 and that (iii) the reactivity at elevated temperatures is better. Compared to UP and epoxy resins the reduced odour nuisance is also advantageous. This facilitates processing and has the result that the polymerizable composition is usable for longer.

The superordinate term "adduct" is to be understood as meaning urethane, thiourethane and urea adducts of a compound of formula (I) with a compound having at least one isocyanate group. A urethane adduct is particularly preferred. The adducts according to the invention are formed when an isocyanate reacts with the functional group B of the compound defined in formula (I). When B is a hydroxyl group a urethane adduct is formed. When B is a thiol group a thiourethane adduct is formed. And when B is NH₂ or NHR⁴ a urea adduct is formed. To the extent that R¹ and/or R² are hydrogen this likewise forms urea adducts.

Contemplated isocyanates for producing the adducts according to the invention in principle include all isocyanates. The choice of suitable isocyanates is not limited to isocyanates having aliphatically, araliphatically and cycloaliphatically bonded isocyanate groups. Isocyanates having aromatically bonded isocyanate groups are likewise employable therefor. Monomeric and oligomeric polyisocyanates are also suitable. Since a suitable isocyanate must comprise at least one isocyanate group monoisocyanates are likewise suitable for producing the adducts according to the invention. It is moreover possible to employ any isocyanate-bearing prepolymer.

In a preferred embodiment of the present invention the isocyanate used for producing the adduct is selected from the group consisting of MDI, TDI, XDI, TXDI, BDI, HDI, PDI, IPDI, oligomerized HDI, oligomerized PDI and oligomerized IPDI, mixtures of the abovementioned isocyanates and reaction products of the abovementioned isocyanates to the extent that these reaction products still contain at least one free isocyanate group.

If the intention is to reduce thermolatent catalysts it is preferable to employ an isocyanate having aliphatically or cycloaliphatically bonded isocyanate groups, more preferably a polyisocyanate having aliphatically bonded isocyanate groups and yet more preferably HDI. Said isocyanates may be in monomeric or oligomeric form. The use of oligomeric aliphatic polyisocyanates, in particular of oligomeric HDI, is very particularly preferred. The study underlying the present invention has shown that adducts of aliphatic isocyanates with compounds of formula (I) exhibit thermolatent behaviour in the crosslinking of both aliphatic and aromatic polyisocyanates.

In a preferred embodiment of the present invention the isocyanate composition used for producing the adduct according to the invention contains at least 20 mol%, preferably at least 50 mol%, more preferably at least 70 mol% yet more preferably at least 80 mol% and most preferably at least 90 mol% of isocyanate groups that are aliphatically or cycloaliphatically bonded. It is particularly preferable when the abovementioned proportions of isocyanate groups are aliphatically bonded. It is very particularly preferable when the isocyanate composition used for producing the adduct according to the invention contains at least 95 mol% of aliphatically bonded isocyanate groups, in particular as a constituent of HDI.

By contrast, if a catalyst achieving very high reaction rates is desired it is preferable to employ adducts with aromatically bonded isocyanate groups. These isocyanates too may be in monomeric or oligomeric form.

The study underlying the present invention has surprisingly shown that adducts which are based on a mixture of isocyanates having aliphatically bonded isocyanate groups and isocyanates with aromatically bonded isocyanate groups have advantageous properties. Adducts which are based on a mixture of the aforementioned polyisocyanate species which comprises at least 50 wt.-% isocyanates with aliphatically bound isocyanate groups and 5 wt.-% to 50 wt.-% isocyanates with aromatically bound isocyanate groups have at most temperatures a viscosity which is lower than the viscosity of an adduct based purely polyisocyanates with aliphatically bound isocyanate groups. At the same time these adducts show an increased reaction rate while maintaining a sufficient pot life at lower temperatures. Thus, such adducts can be easily processed due to their low viscosity and do not need to be added to a reaction mixture in large quantities in order to enable a speedy reaction.

Therefore, in a particularly preferred embodiment, the present invention relates to an adduct of a compound of formula (I) and at least one compound having at least one aliphatically bound isocyanate group and at least one further compound having at least one aromatically bound isocyanate group, wherein the first compound makes up at least 50 wt.-% of all isocyanates used for preparing the adduct and the second compound makes up 5 wt.-% to 50 wt.-% of all isocyanates used for preparing the adduct. More preferred is a range between a weight ratio of 5 : 95 and 35 : 65 (aromatic isocyanate : aliphatic isocyanate). Most preferred is a range between a weight ratio of 5 : 95 and 20 : 80 (aromatic isocyanate : aliphatic isocyanate).

It is preferred that the percentages given above to add up to at least 90 wt.-% of all isocyanates used for preparing the adduct, more preferably they add up to at least 98 wt.-%. Suitable isocyanates with aliphatically bound isocyanate groups are disclosed below in this application. Preferred are monomeric HDI, monomeric PDI, oligomeric HDI and oligomeric PDI. Suitable isocyanates with aromatically bound isocyanate groups are also disclosed below in this application. Preferred is MDI.

"Reaction products of the abovementioned isocyanates" are compounds formed by the reaction of one of the recited isocyanates with a further isocyanate, with an amine, thiol or alcohol or with a combination of an amine, thiol or alcohol and a further isocyanate. Concerned here are amines, thiols and alcohols which do not conform to formula (I). It is essential to the invention that the reaction product still comprises at least one free isocyanate group by means of which it may react with a compound of formula (I) and thus form an adduct according to the invention. Particularly preferred as reaction products are the isocyanate-bearing prepolymers more particularly defined hereinbelow.

### Production of the adduct

In the production of the adduct according to the invention the stoichiometry of free isocyanate groups of the employed isocyanate or of the employed isocyanates and the compound of formula (I) is preferably chosen such that the molar ratio of the functional group B to the free isocyanate groups present is between 0.3 : 1.0 and 1.6 : 1.0, preferably between 0.9 : 1.0 and 1.4 : 1.0.

In a particularly preferred embodiment of the present invention the molar ratio of all isocyanate-reactive groups in the compound of formula (I) to the isocyanate groups of the compound having at least one isocyanate group is at least 1.0 : 1.0 and more preferably between 1.0 : 1.0 and 1.4 : 1.0. This embodiment is characterized in that the finished adduct/the finished catalyst composition no longer comprises any free isocyanate groups. If unreacted isocyanate groups are present in the finished catalyst composition the catalyst brings about during storage a slow crosslinking of these isocyanate groups with one another and thus a viscosity increase of the catalyst composition. If the amount of unreacted isocyanate groups in the finished catalyst composition is too high the viscosity increase can impair the usability of the catalyst composition and can even result in its complete curing so that a mixing of the catalyst composition with isocyanates to be crosslinked is impossible.

Production of the adducts according to the invention may be effected by any processes for producing urethanes, thiourethanes or ureas known to those skilled in the art. It is particularly advantageous when this is effected by slow mixing of the compound of formula (I) and of the employed isocyanate. The reaction generally proceeds by autocatalytic means. Should the reaction rate be insufficient without catalyst addition, the known urethane, thiourethane and urea-forming catalysts may be utilized.

In a preferred embodiment the isocyanate is slowly added to the catalyst optionally with cooling.

In a further preferred embodiment isocyanate and catalyst are quantitatively mixed in an optionally cooled static mixer or reactive mixer and reacted in an optionally cooled reaction tube.

In a further preferred embodiment isocyanate and catalyst are quantitatively mixed and reacted in a cooled static mixer.

It is preferable when the reaction of the catalyst with the isocyanate is carried out at temperatures of not more than 100°C, preferably not more than 80°C, particularly preferably not more than 60°C and very particularly preferably not more than 40°C and preferably under protective gas since this makes it possible to obtain products of optimal colour number. The temperature must be above the freezing point of the particular isocyanate and the reaction is preferably performed at a minimum temperature of 0°C.

### Polyisocyanate

In the present application the term "polyisocyanate" is to be understood as meaning any compound comprising on average at least 1.8, preferably at least 2.0 and particularly preferably 2.1 isocyanate groups. By contrast "monoisocyanate" is to be understood as meaning a compound having on average not more than 1.6 isocyanate groups per molecule, in particular only having one isocyanate group per molecule.

In the present application the term "polyisocyanates" refers to both monomeric and/or oligomeric polyisocyanates. For the understanding of many aspects of the invention, however, it is important to distinguish between monomeric diisocyanates and oligomeric polyisocyanates. Where reference is made in this application to "oligomeric polyisocyanates", this means polyisocyanates formed from at least two monomeric diisocyanate molecules, i.e. compounds that constitute or contain a reaction product formed from at least two monomeric diisocyanate molecules.

### Oligomeric isocyanates

Oligomeric isocyanates are obtained by "modification" of a monomeric isocyanate. "Modification" is to be understood as meaning the reaction of monomeric diisocyanates to afford oligomeric polyisocyanates having a uretdione, isocyanurate, allophanate, biuret, iminooxadiazinedione and/or oxadiazinetrione structure. Preferably employed as reactants for the production of oligomeric isocyanates are diisocyanates.

Thus for example hexamethylene diisocyanate (HDI) is a "monomeric diisocyanate" since it contains two isocyanate groups and is not a reaction product of at least two polyisocyanate molecules:

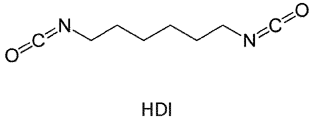

By contrast, reaction products of at least two HDI molecules which still have at least two isocyanate groups are "oligomeric polyisocyanates" in the context of the invention. Proceeding from monomeric HDI representatives of such "oligomeric polyisocyanates" include for example the HDI isocyanurate and the HDI biuret each constructed from three monomeric HDI units:

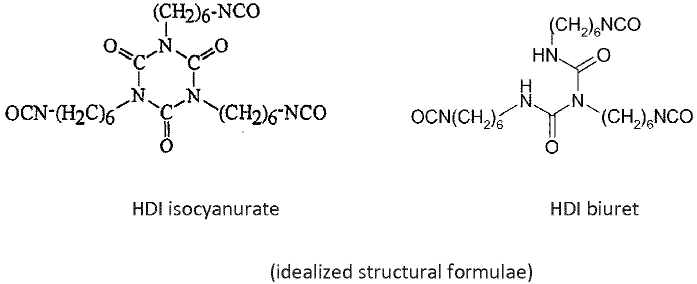

Production processes for oligomeric polyisocyanates having a uretdione, isocyanurate, allophanate, biuret, iminooxadiazinedione and/or oxadiazinetrione structure are described, for example, in J. Prakt. Chem. 336 (1994) 185 - 200, in DE-A 1 670 666, DE-A 1 954 093, DE-A 2 414 413, DE-A 2 452 532, DE-A 2 641 380, DE-A 3 700 209, DE-A 3 900 053 and DE-A 3 928 503 or in EP-A 0 336 205, EP-A 0 339 396 and EP-A 0 798 299.

It is particularly preferable when the monomeric isocyanates defined hereinbelow are used as the starting materials for modification.

The polymerizable composition according to the invention may contain oligomeric and polymeric polyisocyanates in any desired mixing ratios. For reasons of industrial safety, preference is in principle given to polymerizable compositions whose polyisocyanate component, i.e. the entirety of all polyisocyanates present in said composition, consists of oligomeric polyisocyanates to an extent of at least 90% by weight, preferably at least 95% by weight and more preferably at least 98% by weight. However, if desired, for example for reducing the viscosity of a polymerizable composition, the polyisocyanate component may also contain up to 20% by weight or preferably up to 50% by weight of monomeric polyisocyanates.

### Isocyanates having aliphatically bonded isocyanate groups

In an isocyanate having aliphatically bonded isocyanate groups all isocyanate groups are bonded to a carbon atom that is part of an open carbon chain. This may be unsaturated at one or more sites. The aliphatically bonded isocyanate group or - in the case of polyisocyanates - the aliphatically bonded isocyanate groups are preferably bonded at the terminal carbon atoms of the carbon chain.

Polyisocyanates having aliphatically bonded isocyanate groups that are particularly suitable according to the invention are 1,4-diisocyanatobutane (BDI), 1,5-diisocyanatopentane (PDI), 1,6-diisocyanatohexane (HDI), 2-methyl-1,5-diisocyanatopentane, 1,5-diisocyanato-2,2-dimethylpentane, 2,2,4- or 2,4,4-trimethyl-1,6-diisocyanatohexane and 1,10-diisocyanatodecane.

### Isocyanates having cycloaliphatically bonded isocyanate groups

In an isocyanate having cycloaliphatically bonded isocyanate groups all isocyanate groups are bonded to carbon atoms which are part of a closed ring of carbon atoms. This ring may be unsaturated at one or more sites provided that it does not attain aromatic character as a result of the presence of double bonds.

Polyisocyanates having cycloaliphatically bonded isocyanate groups that are particularly suitable according to the invention are 1,3- and 1,4-diisocyanatocyclohexane, 1,4-diisocyanato-3,3,5-trimethylcyclohexane, 1,3-diisocyanato-2-methylcyclohexane, 1,3-diisocyanato-4-methylcyclohexane, 1-isocyanato-3,3,5-trimethyl-5-isocyanatomethylcyclohexane (isophorone diisocyanate; IPDI), 1-isocyanato-1-methyl-4(3)-isocyanatomethylcyclohexane, 2,4'- and 4,4'-diisocyanatodicyclohexylmethane (H12MDI), 1,3- and 1,4-bis(isocyanatomethyl)cyclohexane, bis(isocyanatomethyl)norbornane (NBDI), 4,4'-diisocyanato-3,3'-dimethyldicyclohexylmethane, 4,4'-diisocyanato-3,3',5,5'-tetramethyldicyclohexylmethane, 4,4'-diisocyanato-1,1'-bi(cyclohexyl), 4,4'-diisocyanato-3,3'-dimethyl-1,1'-bi(cyclohexyl), 4,4'-diisocyanato-2,2',5,5'-tetramethyl-1,1'-bi(cyclohexyl), 1,8-diisocyanato-p-menthane, 1,3-diisocyanatoadamantane and 1,3-dimethyl-5,7-diisocyanatoadamantane.

### Isocyanates having araliphatically bonded isocyanate groups

In an isocyanate having araliphatically bonded isocyanate groups all isocyanate groups are bonded to methylene radicals which are in turn bonded to an aromatic ring.

Polyisocyanate having aliphatically bonded isocyanate groups that are particularly suitable according to the invention are 1,3- and 1,4-bis(isocyanatomethyl)benzene (xyxlylene diisocyanate; XDI), 1,3- and 1,4-bis(1-isocyanato-1-methylethyl)benzene (TMXDI) and bis(4-(1-isocyanato-1-methylethyl)phenyl) carbonate.

According to the invention the polymerizable composition may contain any desired mixtures of the abovementioned isocyanates in monomeric and/or oligomeric form.

### Isocyanate having an aromatically bonded isocyanate group

In an isocyanate having aromatically bonded isocyanate groups all isocyanate groups are bonded directly to carbon atoms which are part of an aromatic ring.

Isocyanates having aromatically bonded isocyanate groups that are particularly suitable according to the invention are 2,4- and 2,6-diisocyanatotoluene (TDI), 2,4'- and 4,4'-diisocyanatodiphenylmethane (MDI) and 1,5-diisocyanatonaphthalene.

### Monoisocyanates

Monoisocyanates particularly suitable according to the invention are preferably selected from the group consisting of n-butyl isocyanate, n-amyl isocyanate, n-hexyl isocyanate, n-heptyl isocyanate, n-octyl isocyanate, undecyl isocyanate, dodecyl isocyanate, tetradecyl isocyanate, cetyl isocyanate, stearyl isocyanate, cyclopentyl isocyanate, cyclohexyl isocyanate, 3- or 4-methylcyclohexyl isocyanate, methylbenzyl isocyanate, methyl isocyanate, (trimethylsilyl) isocyanate, 1-naphtyl isocyanate, 3-methyl-2-butyl isocyanate, 1-(4-methoxyphenyl)ethyl isocyanate, 1-(3-methoxyphenyl)ethyl isocyanate, 1-phenylpropyl isocyanate, 2-octyl isocyanate, 2-heptyl isocyanate, 4-butyl-2-methylphenyl isocyanate, 3-(triethoxysilyl)propyl isocyanate, 2-benzyloxycyclohexyl isocyanate, 1-(4-chlorophenyl)ethyl isocyanate, 2-nonyl isocyanate, 1-(4-bromophenyl)ethyl isocyanate, 2,1,3-benzothiadiazol-4-yl isocyanate, p-phenylazophenyl isocyanate, phenyl isocyanate, ethyl isocyanate, chlorosulfonyl isocyanate, allyl isocyanate, benzyl isocyanate, propyl isocyanate, isoproyl isocyanate, furfuryl isocyanate, propyl isocyanate, octadecyl isocyanate, trichloroacetyl isocyanate, benzoyl isocyanate, phenethyl isocyanate, p-tolyl isocyanate, o-tolyl isocyanate, m-tolylisocyanat, 3,4-dimethoxyphenyl isocyanate, 2,4-dimethoxyphenyl isocyanate, 3,5-dimethoxyphenyl isocyanate, 2,5-dimethoxyphenyl isocyanate, tert-butyl isocyanate, 3,5-dimethylphenyl isocyanate, 2,6-dimethylphenyl isocyanate, 4-ethylphenyl isocyanate, 4-methylbenzyl isocyanate, 2-methylbenzyl isocyanate, 3-methylbenzyl isocyanate, 4-methoxyphenyl isocyanate, 4-tert-butylphenyl isocyanate, 2-methoxyphenyl isocyanate, 3,4,5-trimethoxyphenyl isocyanate, 2,4-dimethoxybenzyl isocyanate, 4-phenylbutyl isocyanate, 4-ethylphenethyl isocyanate, 4-methoxybenzyl isocyanate, benzenesulfonyl isocyanate, 2-methoxybenzyl isocyanate, 3-ethoxyphenyl isocyanate, 3-methoxybenzyl isocyanate, 2,2-diphenylethyl isocyanate, 1,1,3,3-tetramethylbutyl isocyanate, 2-ethylhexyl isocyanate, 4-biphenylyl isocyanate, 3-phenylpropyl isocyanate, 2,3-dimethoxyphenethyl isocyanate, decyl isocyanate, cyclohexanemethyl isocyanate, 3,4-methylendioxyphenethyl isocyanate, 3,4-dimethoxyphenethyl isocyanate, 5-indanyl isocyanate, cycloheptyl isocyanate, 2-phenylcyclopropyl isocyanate, 1-cyclohexylethyl isocyanate, 4-nitrophenyl isocyanate, 1-adamantyl isocyanate, 2-nitrophenyl isocyanate, 3-nitrophenyl isocyanate, pyridine-3-isocyanate, chloroacetyl isocyanate, 2,6-diisopropylphenyl isocyanate, hexadecyl isocyanate, 4-acetylphenyl isocyanate, 4-phenoxyphenyl isocyanate, 4-pentylphenyl isocyanate, 3-phenoxyphenyl isocyanate, p-toluenesulfonyl isocyanate, 2-chloroethyl isocyanate, 2-bromophenyl isocyanate, 3-chlorophenyl isocyanate, 2-chlorophenyl isocyanate, 4-bromophenyl isocyanate, 4-chlorophenyl isocyanate, 2-naphthyl isocyanate, 4-fluorophenyl isocyanate, 2-bromoethyl isocyanate, 4-cyanophenyl isocyanate, 3,4-dichlorophenyl isocyanate, 2,3,4-trifluorophenyl isocyanate, 3-cyanophenyl isocyanate, 2,6-dichlorophenyl isocyanate, diethoxyphosphinyl isocyanate, 2,4-dichlorophenyl isocyanate, 3,3,4,4,5,5,6,6,7,7,8,8,9,9,10,10,10-heptadecafluorodecyl isocyanate, 4-fluorobenzyl isocyanate, 2-fluorophenyl isocyanate, 3-chloropropyl isocyanate, 3-fluorophenyl isocyanate, 4-iodophenyl isocyanate, 3,5-dichlorophenyl isocyanate, 4-chlorobenzenesulfonyl isocyanate, 2,4,6-tribromophenyl isocyanate, 2-iodophenyl isocyanate, 3,4-difluorophenyl isocyanate, 3-bromophenyl isocyanate, 2,4-dichlorobenzyl isocyanate, 2,5-difluorophenyl isocyanate, 2-benzylphenyl isocyanate, 2-fluorobenzyl isocyanate, 4-fluorophenethyl isocyanate, pentafluorophenyl isocyanate, 2,4-dichlorophenethyl isocyanate, 4-chlorobenzyl isocyanate, diphenylmethyl isocyanate, tributyltin isocyanate, 2-chlorobenzenesulfonyl isocyanate, 2-chlorobenzyl isocyanate, 3,3-diphenylpropyl isocyanate, 3,4,5-trimethoxybenzyl isocyanate, 3-chlorophenethyl isocyanate, 3-fluorobenzyl isocyanate, 2,6-difluorophenyl isocyanate, 3-iodophenyl isocyanate, 2,4-difluorophenyl isocyanate, 2-cyanophenyl isocyanate, 2-fluorophenethyl isocyanate, 2-thienyl isocyanate, 3,4-dichlorobenzyl isocyanate, 3,4-dichlorophenethyl isocyanate, 4-benzylphenyl isocyanate, 4-bromobenzyl isocyanate, 4-fluorobenzosulfonyl isocyanate, mPEG5K isocyanate, 3,5-dimethylisoxazol-4-yl isocyanate, 2-methoxy-5-methylphenyl isocyanate, 2-(4-biphenyl)ethyl isocyanate, 2-ethyl-6-methylphenyl isocyanate, 2-methyl-5-phenyl-3-furyl isocyanate, 1-(1-naphthyl)ethyl isocyanate, 3,4-(methylenedioxy)phenyl isocyanate, 2,3-dihydro-1-benzofuran-5-yl isocyanate, 4-methoxy-2-nitrophenyl isocyanate, 3,5-bis(trifluoromethyl)phenyl isocyanate, 4-(maleimido)phenyl isocyanate, 4-(dimethylamino)phenyl isocyanate, 3-(trifluoromethyl)phenyl isocyanate, 4-(chlorosulfonyl)phenyl isocyanate, 3-isopropenyl-α,α-dimethylbenzyl isocyanate, 3-chloro-4-methylphenyl isocyanate, 4-(trifluoromethyl)phenyl isocyanate, 2-(trifluoromethyl)phenyl isocyanate, 4,4'-oxybis(phenylisocyanate), 4-(chloromethyl)phenyl isocyanate, 4-chloro-3-(trifluoromethyl)phenyl isocyanate, 9H-fluoren-2-yl isocyanate, 2-(chloromethyl)phenyl isocyanate, 2- fluoro-5-(trifluoromethyl)phenyl isocyanate, 2-fluoro-3-(trifluoromethyl)phenyl isocyanate, 4-(benzyloxy)phenyl isocyanate, 4-fluoro-3-(trifluoromethyl)phenyl isocyanate, 4-fluoro-3-methylphenyl isocyanate, 3-fluoro-5-(trifluoromethyl)phenyl isocyanate, 4-chloro-2-fluorophenyl isocyanate, 5-fluoro-2-methylphenyl isocyanate, 2,3-dimethyl-6-nitrophenyl isocyanate, 2-(trifluoromethoxy)phenyl isocyanate, 2-fluoro-5-methylphenyl isocyanate, 4-(difluoromethoxy)phenyl isocyanate, 4-methyl-2-nitrophenyl isocyanate, 3-fluoro-2-methylphenyl isocyanate, 4-(trifluoromethylthio)phenyl isocyanate, 4-fluoro-2-(trifluoromethyl)phenyl isocyanate, 1-(4-fluorophenyl)ethyl isocyanate, 1-benzothiophen-5-yl isocyanate, 2-(difluoromethoxy)phenyl isocyanate, 2-(thien-2-yl)ethyl isocyanate, 2-bromo-4,6-difluorophenyl isocyanate, 2-chloro-4,6-dimethylphenyl isocyanate, 2-chloro-4-(trifluoromethyl)phenyl isocyanate, 2-chloro-4-(trifluoromethylthio)phenyl isocyanate, 2-chloro-5-methylphenyl isocyanate, 2-fluoro-4-iodophenyl isocyanate, 3-bromo-2,4,6-trimethylphenyl isocyanate, 3-chloro-2-fluorphenyl isocyanate, 3-chloro-2-methylphenyl isocyanate, 4-(trifluoromethyl)benzyl isocyanate, 4-bromo-2,6-difluorophenyl isocyanate, 4-bromo-2,6-dimethylphenyl isocyanate, 4-bromo-2-(trifluoromethyl)phenyl isocyanate, 4-bromo-2-chloro-6-methylphenyl isocyanate, 4-bromo-2-chloro-6-methylphenyl isocyanate, 4-bromo-2-ethylphenyl isocyanate, 4-chloro-2-phenoxyphenyl isocyanate, 4-ethoxy-2-nitrophenyl isocyanate, 4-fluoro-2-nitrophenyl isocyanate, 5-chloro-2-methylphenyl isocyanate, 5-chloro-2-phenoxyphenyl isocyanate, 5-methyl-2-nitrophenyl isocyanate, 5-phenyl-2-thienyl isocyanate, 6-fluoro-4H-1,3-benzodioxin-8-yl isocyanate, 9H-fluoren-9-yl isocyanate, benzyl isocyanate, ethyl isocyanate, trichloroacetyl isocyanate, 1-phenylethyl isocyanate, ethyl isocyanate formate, isocyanatophosphonic dichloride, 2-isocyanatoethyl methacrylate, 3-isocyanato-4-methoxybiphenyl, 2,4,6-trichlorophenyl isocyanate, triphenylsilyl isocyanate, 2,6-dibromo-4-ethylphenyl isocyanate, 2-chloro-4-nitrophenyl isocyanate, 2-tert-butyl-6-methylphenyl isocyanate, 4,4'-methylenebis(2-chlorophenyl isocyanate), 4,5-dimethyl-2-nitrophenyl isocyanate, 4-chloro-2-(trifluoromethyl)phenyl isocyanate, 4-chloro-2-nitrophenyl isocyanate, 1-isocyanato-2,3-dimethoxybenzene, 3-isocyanatopentane, isocyanatocyclobutane, isocyanato(methoxy)methane, ethyl (4-isocyanatophenyl)acetate, ethyl 4-(isocyanatomethyl)cyclohexanecarboxylate, 1,1-dimethoxy-2-isocyanatoethane, 1-chloro-3-fluoro-2-isocyanatobenzene, 2-chloro-3-fluorophenyl isocyanate, 2-isocyanato-3-methylbutyric acid methyl ester, 2-isocyanato-5-methylbenzonitrile, 5-chloro-2-isocyanatobenzonitrile, 5-ethyl-2-isocyanatobenzonitrile, 6-isocyanatohexanoic acid methyl ester, dimethyl 2-isocyanatoterephthalate, ethyl 2-isocyanato-4-methylvalerate, methyl 2-isocyanato-4-(methylsulfanyl)butanoate, methyl 2-isocyanato-4-methylpentanoate, ethyl isocyanatoacetate, phenyl isocyanatoformate, methyl 4-isocyanatobenzoate, methyl 3-isocyanatobenzoate, methyl isocyanatoformate, dimethyl 5-isocyanatoisophthalate and any desired mixtures of such monoisocyanates.

Thioisocyanates are likewise suitable. Preferred thioisocyanates are selected from the group consisting of 4-fluorobenzyl isothiocyanate, dibutyltin diisothiocyanate, 2,6-difluorophenyl isothiocyanate, 3-cyanophenyl isothiocyanate, 3-nitrophenyl isothiocyanate and phenyl isocyanate.

Particular preference is given to monoisocyanates selected from the group consisting of cyclohexyl isocyanate, phenyl isocyanate, octadecyl isocyanate and hexyl isocyanate.

Likewise suitable are mono- or polyisocyanates obtained by the modification of monomeric isocyanates as described hereinabove.

### Prepolymers

Isocyanate-bearing prepolymers suitable for the production of the adducts according to the invention are obtained by reaction of an alcohol, an amine or a thiol with a polyisocyanate. A molar excess of isocyanate groups to isocyanate-reactive groups must be present.

Suitable alcohols are mono- or polyhydric monomeric alcohols, preferably selected from the group consisting of hexanol, butanediol.

The polyether diols and polycarbonate diols known from the prior art are also suitable for producing the adduct according to the invention.

Preferred as the isocyanate for the production of the isocyanate-bearing prepolymer are HDI in monomeric form, oligomerized HDI and mixtures thereof.

### Polymerizable composition

In a further embodiment the present invention relates to a polymerizable composition containing
a) at least one polyisocyanate having isocyanate groups selected from the group consisting of aliphatically, cycloaliphatically, araliphatically and aromatically bonded isocyanate groups; and
b) at least one adduct of a compound according to formula (I) and a compound having at least one isocyanate group 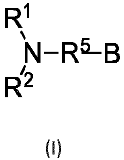
   wherein R¹ and R² are independently of one another selected from the group consisting of hydrogen, methyl, ethyl, propyl, isopropyl, butyl and isobutyl, branched C5-alkyl, unbranched C5-alkyl, branched C6-alkyl, unbranched C6-alkyl, branched C7-alkyl and unbranched C7-alkyl;
   R⁵ is selected from the group consisting of propylene, butylene, pentylene and a radical of formula (II) as defined hereinabove;
   wherein A in formula (II) is selected from the group consisting of O, S and NR³, wherein R³ is selected from the group consisting of H, methyl, ethyl, propyl, isopropyl, butyl und isobutyl, preferably H and methyl; and
   B is independently of A selected from the group consisting of OH, SH NHR⁴ and NH₂, wherein R⁴ is selected from the group consisting of methyl, ethyl and propyl, preferably methyl;
   wherein the ratio of isocyanate groups to isocyanate-reactive groups in the polymerizable composition is at least 2:1.

All definitions given hereinabove for the adduct according to the invention also apply to this embodiment.

In the present application the entirety of all monomeric and oligomeric polyisocyanates present in the polymerizable composition is also referred to as the "polyisocyanate component" of the polymerizable composition.

A "polymerizable composition" is a composition which contains at least the above-defined components and may be cured to afford a polymer by crosslinking of the free isocyanate groups present in the composition. The adduct of the compound of formula (I) acts as a catalyst which brings about the crosslinking of the isocyanate groups.

This crosslinking of at least two isocyanate groups preferably forms isocyanurate and/or uretdione groups. Isocyanurate groups are formed predominantly and uretdione groups are only a byproduct. To the extent that isocyanurate groups are formed it is preferable when three isocyanate groups are crosslinked per isocyanurate group formed.

The quantity ratio of the adduct of the compound of formula (I) on the one hand to the at least one polyisocyanate having isocyanate groups selected from the group consisting of aliphatically, cycloaliphatically, araliphatically and aromatically bonded isocyanate groups on the other hand is chosen such that at temperatures between 80°C and 250°C it is possible to crosslink at least 80% of the free isocyanate groups present within not more than one hour. This condition is preferably met when the weight ratio of the compound of formula (I) to the entirety of the polyisocyanates present in the polymerizable composition is between 1 : 1000 and 1 : 20, more preferably between 1 : 500 and 1 : 20, yet more preferably between 1 : 400 and 1 : 20, most preferably between 1 : 300 and 1 : 20. The calculation of the abovementioned weight ratio uses only the weight fraction of the adduct made up by the compound of formula (I).

Preferably at least 80%, more preferably at least 90%, yet more preferably at least 95% and particularly preferably at least 98% of the isocyanate groups present in the polymerizable composition are aliphatically and/or cycloaliphatically bonded.

It is particularly preferable when the polyisocyanate component of the polymerizable composition consists to an extent of at least 80% by weight of at least one polyisocyanate selected from the group consisting of monomeric HDI, oligomeric HDI, monomeric PDI, oligomeric PDI, monomeric IPDI and oligomeric IPDI. It is very particularly preferable when it consists to an extent of at least 90% by weight of at least one of the abovementioned polyisocyanates.

The molar ratio of isocyanate groups to isocyanate-reactive groups in the polymerizable composition is preferably at least 5:1 and more preferably at least 10:1. In the context of the present application "isocyanate-reactive groups" is to be understood as meaning hydroxyl groups, thiol groups and amino groups of primary and secondary amines.

### Kit

In yet a further embodiment the present invention relates to a polymerizable composition containing
a) at least one polyisocyanate having isocyanate groups selected from the group consisting of aliphatically, cycloaliphatically, araliphatically and aromatically bonded isocyanate groups; and
b) at least one adduct of a compound according to formula (I) and a compound having at least one isocyanate group.

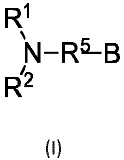

The radicals of the compound of formula (I) and the construction of the adduct were defined hereinabove.

The presence of the two components as a kit means that both components are present together but in separate containers. In a preferred embodiment the kit further contains a user manual which describes the use according to the invention. It is preferable when the kit contains the component b) in an amount which is suitable for crosslinking at least 80% of the isocyanate components present in the kit at a temperature between 80°C to 250°C in not more than 10 minutes.

The polyisocyanate present in the kit according to the invention corresponds to the "polyisocyanate component" in the above-defined polymerizable composition. All definitions provided there thus also apply to the polyisocyanate present in the kit.

### Use

The present invention also relates to the use of at least one adduct of a compound of formula (I) as defined above and a compound having at least one isocyanate group 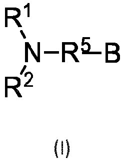 for crosslinking at least two isocyanate groups selected from the group consisting of aliphatically, cycloaliphatically, araliphatically and aromatically bonded isocyanate groups.

All definitions recited above for the polymerizable composition also apply to this embodiment unless explicitly defined otherwise.

It is preferable when the crosslinking of the at least two isocyanate groups selected from the group consisting of aliphatically, cycloaliphatically, araliphatically and aromatically bonded isocyanate groups forms an isocyanurate group.

In a particularly preferred embodiment the adduct of the compound of formula (I) is used as a thermolatent catalyst.

Particular preference is given to the use of at least two aliphatically and/or cycloaliphatically bonded isocyanate groups. It is very particularly preferable when the isocyanate groups present in at least one compound selected from the group consisting of HDI, PDI, IPDI, oligomeric HDI, oligomeric PDI and oligomeric IPDI are crosslinked with one another.

The use according to the invention preferably results in a highly crosslinked polymer. In the context of the present invention chemically highly crosslinked polymers are to be understood as meaning those having an average chemical network arc length Mc of not more than 1000 g/mol, preferably not more than 500 g/mol, particularly preferably not more than 400 g/mol and very particularly preferably 300 g/mol. The average network arc length is defined as the number-average molar mass between the network nodes in a polymer network.

The average network arc length and the crosslinking density may be calculated via swelling measurements according to the HERMANS-FLORY-WALL method in suitable solvents or by measurement of the elastic modulus in the melt in the linear-elastic range at low frequencies, see also John d. Ferry: Viscoelastic properties of polymers 3rd Edition, 1980.

The network arc length is preferably determined by rheological measurement.

In the context of the present invention highly crosslinked materials are to be understood as also meaning those having a storage shear modulus in the melt measured in the linear range of at least 3 ×10⁶ Pa, preferably at least 5 ×10⁶ Pa and very particularly preferably at least 8 × 10⁶ Pa.

The use as a thermolatent catalyst is characterized in that the catalyst is mixed with the isocyanate to be crosslinked and the thus-formed reaction mixture is initially stored at a temperature at which the catalyst shows no significant catalytic activity. The temperature is subsequently raised to a value at which the catalyst is active, thus commencing the crosslinking reaction. Storage is preferably carried out at temperatures of not more than 40°C, more preferably not more than 30°C. The storage duration is measured such that the viscosity of the reaction mixture increases by not more than 200% over this period. At storage temperatures of 30°C this is preferably a period of 30 minutes to 5 days, more preferably of 30 minutes to 24 hours. To activate the catalyst the temperature is raised to 50°C to 250°C, preferably to 80°C to 250°C and more preferably to 120°C to 250°C.

The present invention further relates to the use of a polymerizable composition as defined hereinabove or of a kit as defined hereinabove for producing a polymer.

Said polymer is preferably the matrix material of a composite material. It is particularly preferably the matrix material of a highly filled composite material.

The term "composite material" is well known to those skilled in the art and in principle concerns materials where a filler is embedded in a matrix. According to the invention this matrix is a polymer formed by the crosslinking of the isocyanate groups present in the polyisocyanate component a).

The filler may be any organic or inorganic filler known to those skilled in the art. It may have any desired geometry. However, it is preferably a fibrous organic or inorganic filler.

The aspect ratio of a fibrous filler is greater than 1000, preferably greater than 5000, more preferably greater than 10 000 and most preferably greater than 50 000. The aspect ratio is defined as the length of the fibres divided by the diameter.

While complying with the above-defined aspect ratio the fibrous fillers preferably have a minimum length of 1 m, particularly preferably 50 m and very particularly preferably 100 m.

Preferred inorganic fibres are glass fibres, basalt fibres, boron fibres, ceramic fibres, whiskers, silica fibres and metallic reinforcing fibres. Preferred organic fibres are aramid fibres, carbon fibres, carbon nanotubes, polyester fibres, nylon fibres and Plexiglass fibres. Preferred natural fibres are flax fibres, hemp fibres, wood fibres, cellulose fibres and sisal fibres.

The ratio of the proportions of filler and polymer matrix in the composite material is described as the filler loading. Highly filled systems feature a weight fraction of the filler between 50% by weight and 90% by weight, preferably between 60% by weight and 85% by weight and yet more preferably between 70% by weight and 85% by weight.

### Process

In yet a further embodiment the present invention relates to a process for producing a polymer containing the steps of
a) mixing at least one polyisocyanate having isocyanate groups selected from the group consisting of aliphatically, cycloaliphatically, aromatically and araliphatically bonded isocyanate groups with at least one adduct of a compound of formula (I) and a compound having at least one isocyanate group; 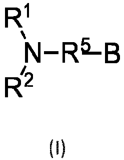
b) curing the polymerizable composition obtained in process step a) by raising the temperature to at least 50°C,
wherein at commencement of process step b) the ratio of isocyanate groups to isocyanate-reactive groups in the polymerizable composition is at least 2:1.

All definitions of the polyisocyanates employable according to the invention, the radicals of the compound of formula (I) and the structure of the adduct of the compound of formula (I) given hereinabove also apply to these embodiments.

The crosslinking of the component in process step a) may be effected using any suitable processes known to those skilled in the art. Process step a) is preferably performed at temperatures of not more than 40°C. Present at the end of process step a) is a reaction mixture which corresponds to the polymerizable composition disclosed at the beginning of the present application. Accordingly, when regarded in isolation, process step a) discloses a process for providing the polymerizable composition according to the invention.

Suitable quantity ratios of the components are described hereinabove in connection with the polymerizable composition. The ratio of isocyanate groups to isocyanate-reactive groups in the polymerizable composition is in particular at least 5:1 and more preferably at least 10:1. The term "isocyanate-reactive groups" is to be understood as meaning hydroxyl, thiol and amino groups.

Since the compound of formula (I) and the adducts thereof are thermolatent catalysts, process step b) is preferably initiated by elevating the temperature of the polymerizable composition. The elevated temperature is preferably also maintained over the total duration of process step b). It is preferable when a temperature of at least 50°C is maintained during the entirety of process step b). The temperature during process step b) is more preferably between 50°C and 250°C, yet more preferably between 80°C and 250°C and most preferably between 120°C and 250°C.

Since the compound of formula (I) and its adducts show no significant catalytic activity at temperatures up to 30°C the polymerizable composition obtained in process step a) may before commencement of process step b) be stored for at least 30 minutes, preferably at least 1 hour and more preferably at least 3 hours while complying with this temperature limit without the viscosity thereof increasing by more than 200%. This storage may have a duration of up to 24 hours. When using adducts having aliphatic and/or cycloaliphatic isocyanates this storage may also be carried out at temperatures of up to 40°C.

The "curing" of the polymerizable composition is effected by crosslinking of the isocyanate groups present in the polyisocyanate. This forms a solid polymer network. Since the compound of formula (I) and its adducts especially catalyze the formation of isocyanurate groups it is preferable when isocyanurate groups are formed and to an extent of at least 80 mol% while the sum of uretdione, urethane, allophanate, urea, biurets, iminooxadiazinedione and oxadiazinetrione structures formed during the curing is below 20 mol%.

Process step b) is complete when the liquid polymerizable composition has formed a solid which retains its shape without external supports such as casting moulds for example. This is preferably the case when at least 75%, more preferably at least 80%, yet more preferably at least 85% and most preferably at least 90% of the isocyanate groups present in the polymerizable composition at commencement of process step b) have been consumed. At temperatures between 80°C and 250°C this state is preferably achieved after not more than 20 minutes.

When production of the composite material is intended the polymerizable composition must contain a filler at commencement of process step b). Said filler may be introduced into the polymerizable composition in various ways. The polymerizable composition as is present at the end of process step a) may be mixed with the filler. However, it is also possible to initially mix with the filler a polyisocyanate having isocyanate groups selected from the group consisting of aliphatically, cycloaliphatically, araliphatically and aromatically bonded isocyanate groups before it is employed in process step a). Suitable fillers are described hereinabove. Process step b) thus affords a composite material where the cured reaction mixture forms a polymer matrix in which the filler has been embedded.

In a preferred embodiment of the invention the filler is a fibrous filler selected from the group consisting of glass fibres, basalt fibres, carbon fibres and mixtures thereof. The fibres may be in loose form but may also have been woven or knitted in any form known to those skilled in the art to form mats or tiles. It is preferable when less than 50% by weight, more preferably less than 35% by weight, yet more preferably less than 20% by weight and most preferably less than 10% by weight of the fibres used are in the form of mats or tiles.

In a particularly preferred embodiment of the present invention the process according to the invention for producing a composite material is a pultrusion process.

Pultrusion is a continuous production process for manufacturing fibre-reinforced plastics profiles. The basic construction of a pultrusion system consists of the fibre rack, apparatuses for fibre guiding, an impregnation means, a curing mould, reciprocal pulling apparatuses and a cutting unit.

The reels of rovings are stored in the fibre rack. From the fibre rack, the fibre rovings are guided via fibre guides to the impregnation means where the fibres are wetted with the reaction mixture from process step a). The fibres are generally already aligned or pre-sorted according to the subsequently desired profile shape via the fibre guides or else the impregnation means. Particularly suitable are fibres which while complying with the aspect ratio defined above have a minimum length of at least 50 m.

Mats, weaves, NCFs or nonwovens may also be integrated into the process if required in order to optimize the mechanical properties for the desired use. Impregnation of the fibres with the reaction mixture may be effected by any methods known to those skilled in the art in the context of the pultrusion process.

The resin-impregnated fibres subsequently pass through the shape-conferring curing mould where the crosslinking of the reactive groups of the resin to form the polymer (matrix) is effected by elevated temperature. This is process step b) of the process. This is frequently followed by a cooling zone, for example air cooling, before the now complete semifinished product is pulled through the alternating pulling devices (pullers). These ensure continuous transport of the material throughout the pultrusion process. In the last process step, the material is cut to the desired length. This is frequently done using a 'flying saw', meaning that the saw runs at the same speed as the material and in so doing cuts it. In this way, a straight cut edge is obtained, and the profile is prevented from backing up and the process is prevented from being stopped during the sawing step.

The present application further relates to a polymer obtainable by the above-described process.

Compared to the catalysts described in WO 2018/054776 A use of the catalyst according to the invention provides a number of advantages.

The amine catalysts according to the invention preferably form a clear solution in known aliphatic isocyanates. The amine catalysts according to the invention are not ionic compounds and thus do not require elaborate stabilization to prevent clouding of the reaction mass after polymerization. Due to the absence of ionic compounds the catalysts according to the invention do not impair the electrical properties of the reaction product in respect of dielectric strength.

### Figures

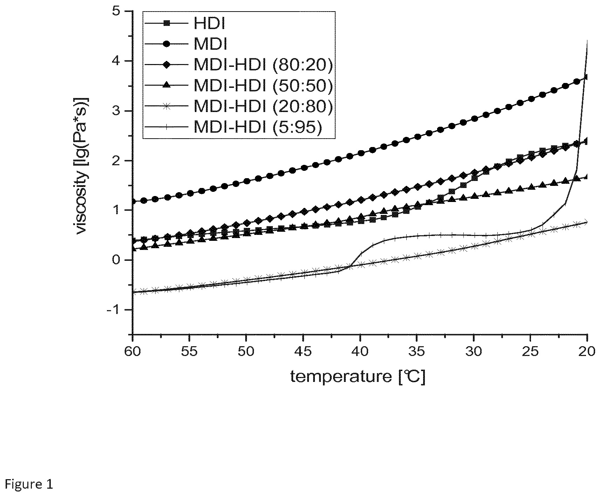

**Figure 1** shows the viscosities of the adducts of example 42 at different temperatures
The working examples which follow serve merely to illustrate the invention. They are not intended to limit the scope of protection of the patent claims in any manner.

### Working examples

### General details:

All percentages, unless stated otherwise, are based on percent by weight (% by weight).

The ambient temperature of 23°C at the time of conducting the experiments is referred to as RT (room temperature).

The methods detailed hereinafter for determining the relevant parameters were employed for performing/evaluating the examples and are also the methods for determining the parameters relevant in accordance with the invention in general.

### Determination of phase transitions by DSC

The phase transitions were determined by means of DSC (differential scanning calorimetry) with a Mettler DSC 12E (Mettler Toledo GmbH, Giessen, Germany) in accordance with DIN EN 61006. Calibration was effected via the melt onset temperature of indium and lead. 10 mg of substance were weighed out in standard capsules. The measurement was effected by three heating runs from - 50°C to +200°C at a heating rate of 20 K/min with subsequent cooling at a cooling rate of 320 K/min. Cooling was effected by means of liquid nitrogen. The purge gas used was nitrogen. The values reported are in each case based on evaluation of the 2nd heating curve. The melting temperatures Tₘ were obtained from the temperatures at the maxima of the heat flow curve. The glass transition temperature T_{g} was obtained from the temperature at half the height of a glass transition step.

### Determination of infrared spectra

The infrared spectra were measured on a Bruker FT-IR spectrometer equipped with an ATR unit.

### Dynamic mechanic analysis (DMA

The temperature at which crosslinking started was determined by DMA. Glass fiber tissue was wetted with the reaction mixture and tested with an amplitude of 200 µ, a heating rate of 2 k per minute and an excitation frequency of 2 Hz. The temperatures given in table 5 indicate the onset of crosslinking.

### Starting compounds

Polyisocyanate A1 is an HDI trimer (NCO functionality > 3) having an NCO content of 23.0% by weight from Covestro AG. The viscosity is about 1200 mPa·s at 23°C (DIN EN ISO 3219/A.3).

Polyisocyanate A2 is HDI having an NCO content of 49.7% by weight from Covestro AG.

Polyisocyanate A3 is IPDI having an NCO content of 37.5% by weight from Covestro AG.

Polyisocyanate A4 is H12MDI having an NCO content of 31.8% by weight from Covestro AG.

Monoisocyanate A5 is cyclohexyl isocyanate and was obtained in a purity of ≥98% from Sigma-Aldrich.

Monoisocyanate A6 is phenyl isocyanate and was obtained in a purity of ≥98% from Sigma-Aldrich.

Monoisocyanate A7 is octadecyl isocyanate and was obtained in a purity of ≥98% from Sigma-Aldrich.

Monoisocyanate A8 is hexyl isocyanate and was obtained in a purity of ≥97% from Sigma-Aldrich.

Polyisocyanate A9 is 1,3-bis(isocyanatomethyl)benzene having an NCO content of 44% by weight from Covestro AG.

Polyisocyanate A10 is 2,4'-diphenylmethane diisocyanate having an NCO content of 33.6% by weight from Covestro AG.

Polyisocyanate A11 is a mixture of 2,4- and 2,6-tolylene diisocyanate in a ratio of 80 : 20 having an NCO content of 48% by weight from Covestro AG.

Polyisocyanate A12 is 4,4'-diphenylmethane diisocyanate having an NCO content of 33.6% by weight from Covestro AG.

Polyisocyanate A13 is a polyisocyanate based on diphenylmethane diisocyanate having an NCO content of 31.5% by weight from Covestro AG. The viscosity is about 90 mPa·s at 25°C (DIN EN ISO 3219/A.3).

K1: N,N,N'-trimethylaminoethylethanolamine having an OH number of 384 mg KOH/g was obtained from Huntsman Corporation.

K2: 2-(2-dimethylaminoethoxy)ethanol having an OH number of 421 mg KOH/g was obtained from Huntsman Corporation.

K3: Benzyldimethylamine was obtained from Huntsman Corporation.

K4: 2,2'-dimorpholine diethyl ether was obtained from Huntsman Corporation.

K5: N-(3-dimethylaminopropyl)-N,N-diisopropanolamine having an OH number of 514 mg KOH/g was obtained from Huntsman Corporation.

K6: Pentamethyldiethylenetriamine was obtained from Covestro AG.

K7: N,N,N'-trimethyl-N'-hydroxyethylbisaminoethyl ether having an OH number of 295 mg KOH/g was obtained from Huntsman Corporation.

K8: N,N,N',N",N"-pentamethyldipropylenetriamine was obtained from Huntsman Corporation.

K9: N,N-bis(3-dimethylaminopropyl)-N-isopropanolamine having an OH number of 229 mg KOH/g was obtained from Huntsman Corporation.

K10: N'-(3-(dimethylamino)propyl)-N,N-dimethyl-1,3-propanediamine was obtained from Huntsman Corporation.

K11: A mixture of 15% bis[(dimethylamino)methyl]phenol and 2,4,6-tris(dimethylaminomethyl)phenol was obtained from Evonik Industries AG.

K12: Sodium {/V-methyl[(2-hydroxy-5-nonylphenyl)methyl]amino}acetate dissolved in glycol was obtained from Evonik Industries AG.

K13: Tris(dimethylaminopropyl)hydrotriazine was obtained from Evonik Industries AG.

All raw materials except for the catalyst were degassed under vacuum before use.

### Production of the catalyst adducts

The isocyanate was added dropwise to the catalyst K1/K2 with cooling and the mixture was then stirred until homogeneous and until residual isocyanate was no longer detectable by IR analysis.

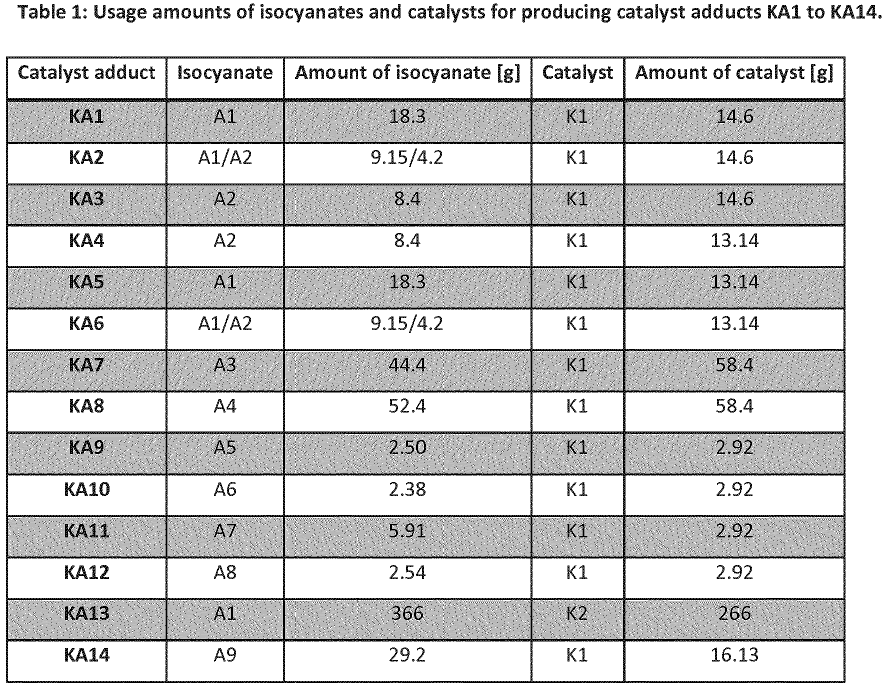

### Production of catalyst adducts using solvent

The isocyanate in ethyl acetate was added dropwise to the catalyst K1 in ethyl acetate with cooling and the mixture was then stirred until homogeneous and until residual isocyanate was no longer detectable by IR analysis. The solvent was then removed under reduced pressure.

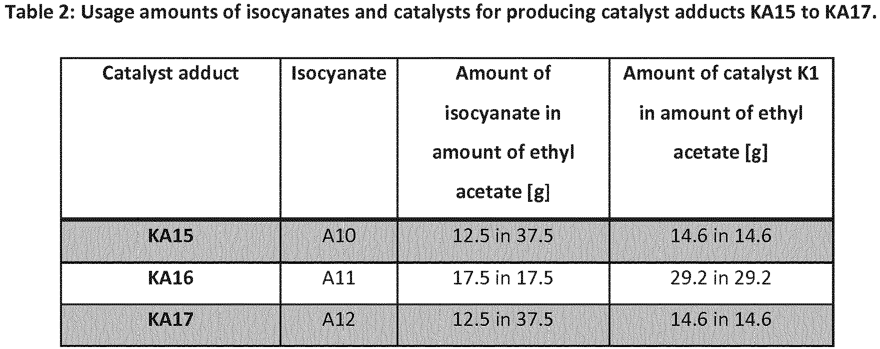

### Production of the reaction mixture

Unless otherwise stated the reaction mixture was produced by mixing polyisocyanate (A1) with a corresponding amount of catalyst and additive at 23°C in a Speedmixer DAC 150.1 FVZ from Hauschild at 2750 min⁻¹. Without any further treatment said mixture was then poured into a suitable mould for crosslinking and cured.

### Working examples 1-17

The amounts of polyisocyanate A1 reported in table 1, catalyst adduct and in each case 0.5 g of zinc stearate were treated in accordance with the abovementioned production procedure for reaction mixtures. Curing in an oven was performed at 220°C over 5 min. The T_{g} of the cured reaction mixtures was 81 - 113°C. The viscosities of the inventive reaction mixtures comprising polyisocyanate A1 (examples 1 and 3) were 1.27 - 1.30 Pa·s directly after production and in both cases increased to 1.36 Pa·s over 4 h.

The working examples 1 to 17 show that these catalysts may be reacted with various isocyanates to form adducts which, in turn, are all catalytically active.

### Comparative examples 18 - 28

The amounts of polyisocyanate A1 reported in table 2, catalyst and in each case 0.5 g of zinc stearate were treated in accordance with the abovementioned production procedure for reaction mixtures. Curing in an oven was performed at 220°C over 5 min. The comparative examples show that various other amine-based catalysts do not result in solid materials under the same curing conditions.

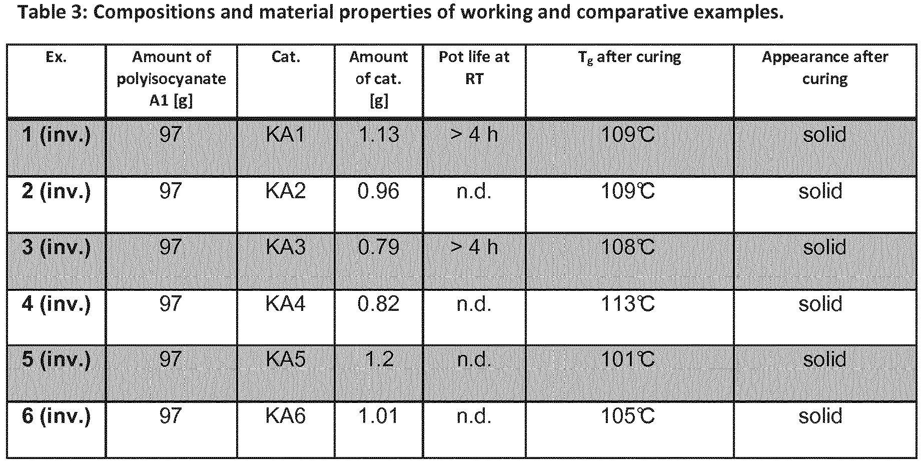

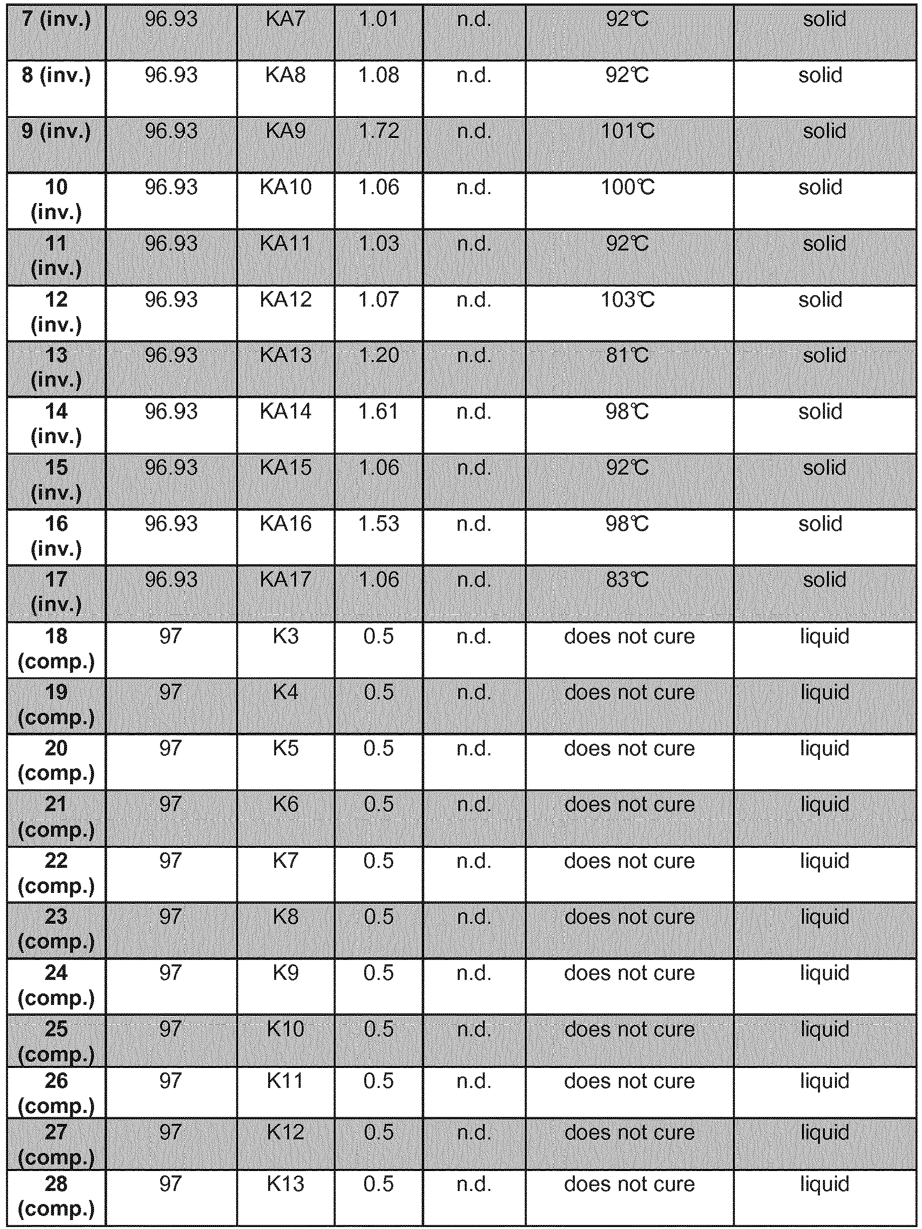

### Working examples 29 - 37 for identifying further suitable compounds for producing adducts

The catalytic activity of the compounds was determined with an n-hexyl isocyanate as the model substrate. The most quantitatively significant reaction product was a trimer. The reaction of the NCO groups was verified by ¹³C-NMR at 100 MHz. The solvent used for the samples was deuterochloroform, its non-deuterated fraction serving as internal standard.

Compounds K14 to K16 were tested.
K14: 3-(dimethylamino)-propanol
K15: 4-(dimethylamino)-butanol
K16: 5-(dimethylamino)-pentanol

n-Hexylisocyanate was in each case admixed with the concentrations of the compounds K14, K15 and K6 reported in the table which follows. Incubation was carried out under the specified conditions

**Table 4:**

| Experiment | Compound | Reaction parameters | Result |
|---|---|---|---|
| 29 | 3-(dimethylamino)-propanol | 80°C for 2 h, 20 mol% | Trimerization detectable but residual NCO content |
| 30 | 3-(dimethylamino)-propanol | 150°C for 5 min, 20 mol% | Trimerization detectable but residual NCO content |
| 31 | 3-(dimethylamino)-propanol | 150°C for 5 min, 0.5 mol% | No reaction detectable |
| 32 | 4-(dimethylamino)-butanol | 80°C for 2 h, 20 mol% | Complete reaction of NCO groups |
| 33 | 4-(dimethylamino)-butanol | 150°C for 5 min, 20 mol% | Complete reaction of NCO groups |
| 34 | 4-(dimethylamino)-butanol | 150°C for 5 min, 0.5 mol% | Complete reaction of NCO groups |
| 35 | 5-(dimethylamino)-pentanol | 80°C, 2 h, 20 mol% | No reaction detectable |
| 36 | 5-(dimethylamino)-pentanol | 150°C, 5 min, 20 mol% | Complete reaction of NCO groups |
| 37 | 5-(dimethylamino)-pentanol | 150°C, 5 min, 0.5 mol% | No reaction detectable |

The experiment shows that alkylene radicals without heteroatoms are also suitable as radical R⁵ to the extent that they contain 3 to 5 carbon atoms. Radicals R⁵ made of 4 carbon atoms are optimal.

Corresponding compounds are of course also suitable starting materials for the production of the inventive adducts.

### Inventive examples 38 to 41 for investigating the effect of isocyanate on activity of catalyst

It was to be investigated whether the structure of the polyisocyanate used for producing the adduct (aliphatic or aromatic) has an effect on the catalytic activity of the adduct.

The INT- 1940 RTM demoulding agent was obtained from Axel Plastics Research Laboratories, INC. and according to the datasheet is a mixture of organic fatty acids and esters.

### Production of the reaction mixture

Unless otherwise stated the reaction mixture was produced by mixing the polyisocyanate with a corresponding amount of catalyst and additive at 23°C in a Speedmixer DAC 150.1 FVZ from Hauschild at 2750 min⁻¹. Without any further treatment said mixture was then poured into a suitable mould for crosslinking and cured. Part of the mixture was subsequently used to carry out investigations on pot life.

### Working example 38

A resin mixture composed of isocyanate A1 (2.47 g), polyisocyanate A13 (22.23 g) and catalyst KA1 (0.30 g) was produced. Curing in the oven for 5 min at 220°C afforded a solid material having a T_{g} of over 200°C. The gel time of the resin mixture at room temperature was more than 22 hours. After 24 h of storage at room temperature a liquid material having a gelled top was obtained.

### Working example 39

A resin mixture composed of isocyanate A1 (5.00 g), polyisocyanate A13 (19.38 g) and catalyst KA1 (0.63 g) was produced. Curing in the oven for 60 min at 100°C afforded a solid material having a T_{g} of more than 280°C. Thermal curing reduced the height of the characteristic NCO band between 2300 to 2250 cm⁻¹ by at least 80%. The gel time of the resin mixture at room temperature was more than 22 h. After 24 h of storage at room temperature a liquid material having a gelled top was obtained.

### Working example 40

A resin mixture composed of isocyanate A1 (3.50 g), polyisocyanate A13 (16.20 g) and catalyst KA1 (0.4 g) was produced. Curing in the oven for 10 min at 100°C afforded a solid material having a T_{g} of more than 280°C. Thermal curing reduced the height of the characteristic NCO band between 2300 to 2250 cm⁻¹ by at least 80%. The gel time of the resin mixture at room temperature was more than 22 h. After 24 h of storage at room temperature a liquid material having a gelled top was obtained.

### Working example 41

A resin mixture composed of isocyanate A1 (96.30 g), INT - 1940 RTM (2.50 g) and catalyst KA16 (1.2 g) was produced. The material became hot and then solid over one minute, thus forming a material having a T_{g} of 101°C. Exothermic curing reduced the height of the characteristic NCO band between 2300 to 2250 cm⁻¹ by at least 80%.

Examples 38 to 40 show that adducts produced with aliphatic polyisocyanates are very good thermolatent catalysts. Reaction mixtures containing said catalysts are storable for more than 20 hours at room temperature without viscosity increase impairing the processability of the mixture. This was surprisingly also found for reaction mixtures having high proportions of aromatic polyisocyanates which are very reactive in combination with conventional catalysts. Nevertheless, a rapid curing of the reaction mixture is possible at elevated temperature. Thus example 38 required only 5 minutes at 220°C for curing.

By contrast, if an aromatic polyisocyanate is used for producing the adduct even normally rather slowly reacting aliphatic polyisocyanates (example 41) undergo a very rapid reaction.

The catalytic properties of the inventive adducts can accordingly be controlled through the choice of a suitable polyisocyanate. Reaction systems which react spontaneously and very rapidly at room temperature as well as systems having a very pronounced thermolatency are obtainable.

### Working example 42: Properties of adducts based on mixtures of aromatic and aliphatic isocyanates

The adducts were prepared as described above. They were derived from K1. Mixtures of HDI and MDI in different proportions were used instead of pure isocyanates. Viscosity at different temperatures and pot life at 23 °C were determined. Onset of crosslinking was determined by DMA. The results are summarized in table 5 below. Pot life was determined at 23 °C. It is defined as the time in which viscosity of the reaction mixture doubled.

The reaction mixture consisted of polyisocyanate A1 and the respective adduct. The concentration of the adduct was adjusted so that the concentration of the amine as core was always 0.5 wt.-%. The concentration of the adduct thus differed as MDI and HDI have different molecular weights and are present in different proportions.

**Table 5:**

| | Viscosity at 20 °C [mPa*s] | Viscosity at 60 °C [mPa*s] | Pot life | Onset of crosslinking [°C] |
|---|---|---|---|---|
| MDI | 4,750,000 | 15,000* | >4h | 98.2 |
| MDI-HDI (80 : 20) | 257,000 | 2,350 | 134 min. | 94.3 |
| MDI-HDI (50 : 50) | 46,800 | 1,650 | 62 min. | 90.4 |
| MDI-HDI (20 : 80) | solid | 227 | >6h | 107.6 |
| MDI-HDI (5 : 95) | solid | 219 | >6h | 136.8 |
| HDI | solid | 2,460 | >4h | 120.0 |

| | | | | |
|---|---|---|---|---|
| *inaccurate measurement as adduct had a high viscosity which prevented easy mixing | | | | |

It can be seen from table 5 as well as figure 1 that at 60 °C adducts which comprise between 5 wt.-% and 50 wt.-% MDI have a lower viscosity than adducts prepared with pure HDI or pure MDI. These adducts retain a pot life of at least one hour while having a low crosslinking temperature which is still practically useful.

## Claims

1. Adduct of a compound according to formula (I) and a compound having at least one isocyanate group 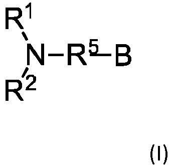
wherein R¹ and R² are independently of one another selected from the group consisting of hydrogen, methyl, ethyl, propyl, isopropyl, butyl, isobutyl, branched C5-alkyl, unbranched C5-alkyl, branched C6-alkyl, unbranched C6-alkyl, branched C7-alkyl and unbranched C7-alkyl;
R⁵ is selected from the group consisting of propylene, butylene, pentylene and a radical of formula (II), preferably from butylene and the radical of formula (II); 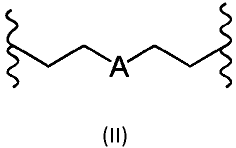
wherein A in formula (II) is selected from the group consisting of O, S and NR³, wherein R³ is selected from the group consisting of hydrogen, methyl, ethyl, propyl, isopropyl, butyl and isobutyl; and
B is independently of A selected from the group consisting of OH, SH NHR⁴ and NH₂, wherein R⁴ is selected from the group consisting of methyl, ethyl and propyl, wherein the adduct is **characterized in that** it no longer comprises any free isocyanate-reactive groups.

2. The adduct according to Claim 1, wherein in formula (II) A is NR³ and R³ is selected from the group consisting of hydrogen, methyl, ethyl, propyl, isopropyl, butyl and isobutyl.

3. The adduct according to Claim 1, wherein in formula (II) A is oxygen.

4. The adduct according to Claim 1, wherein R⁵ is butylene.

5. The adduct according to any of Claims 1 to 4, wherein the isocyanate is a monomeric or oligomeric isocyanate having aliphatically or cycloaliphatically bonded isocyanate groups.

6. The adduct according to any of claims 1 to 5, comprising at least one compound having at least one aliphatically bound isocyanate group and at least one further compound having at least one aromatically bound isocyanate group, wherein the first compound makes up at least 50 wt.-% of all isocyanates used for preparing the adduct and the second compound makes up 5 wt.-% to 50 wt.-% of all isocyanates used for preparing the adduct.

7. Use of an adduct according to any of Claims 1 to 6 as a catalyst for crosslinking at least two isocyanate groups selected from the group consisting of aliphatically, cycloaliphatically, araliphatically and aromatically bonded isocyanate groups.

8. The use as according to Claim 7, wherein the use results in a highly crosslinked polymer.

9. Polymerizable composition containing
a) at least one polyisocyanate having isocyanate groups selected from the group consisting of aliphatically, cycloaliphatically, araliphatically and aromatically bonded isocyanate groups; and
b) at least one adduct according to any of Claims 1 to 6;
wherein the molar ratio of isocyanate groups to isocyanate-reactive groups in the polymerizable composition is at least 2:1.

10. The polymerizable composition according to Claim 9, wherein the polyisocyanate component of the polymerizable composition consists to an extent of at least 80% by weight of at least one polyisocyanate selected from the group consisting of monomeric HDI, oligomeric HDI, monomeric PDI, oligomeric PDI, monomeric H12MDI, oligomeric H12MDI, monomeric IPDI and oligomeric IPDI.

11. Kit containing
a) at least one polyisocyanate having isocyanate groups selected from the group consisting of aliphatically, cycloaliphatically, araliphatically and aromatically bonded isocyanate groups and
b) at least one adduct according to any of Claims 1 to 6.

12. The use of the polymerizable composition according to Claims 9 or 10 or of the kit according to Claim 11 for producing a polymer.

13. Process for producing a polymer containing the steps of
a) mixing at least one polyisocyanate having isocyanate groups selected from the group consisting of aliphatically, cycloaliphatically, araliphatically and aromatically bonded isocyanate groups with an adduct according to any of Claims 1 to 6; and
b) curing the polymerizable composition obtained in process step a) by raising the temperature to at least 50°C,
wherein at commencement of process step b) the ratio of isocyanate groups to isocyanate-reactive groups in the polymerizable composition is at least 2:1.

14. The process according to Claim 13, wherein a period of at least 30 minutes elapses between the end of process step a) and commencement of process step b).

15. The process according to Claim 13 or 14, **characterized in that** the reaction mixture obtained in process step a) is mixed with an organic or inorganic filler before performance of process step b).

16. The process according to Claim 15, **characterized in that** the organic or inorganic filler consists of fibres having a minimum length of 50 m and the curing in process step b) is carried out in a heated mould which imparts the fibre bundle wetted with the reaction mixture with a profile and stabilizes this profile by the curing of the reaction mixture.

17. A polymer obtainable by the process according to Claim 13 or 14 or to a composite material obtainable by the process according to Claim 15 or 16.

## Patentansprüche

1. Addukt einer Verbindung gemäß Formel (I) und einer Verbindung mit wenigstens einer Isocyanatgruppe 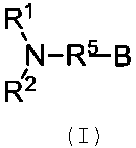
wobei R¹ und R² unabhängig voneinander ausgewählt sind aus der Gruppe bestehend aus Wasserstoff, Methyl, Ethyl, Propyl, Isopropyl, Butyl, Isobutyl, verzweigtem C5-Alkyl, unverzweigtem C5-Alkyl, verzweigtem C6-Alkyl, unverzweigtem C6-Alkyl, verzweigtem C7-Alkyl und unverzweigtem C7-Alkyl;
R⁵ ausgewählt ist aus der Gruppe bestehend aus Propylen, Butylen, Pentylen und einem Rest gemäß Formel (II), bevorzugt aus Butylen und dem Rest gemäß Formel (II); 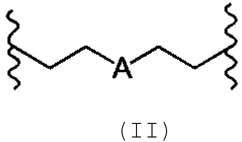
wobei A in Formel (II) ausgewählt ist aus der Gruppe bestehend aus O, S und NR³, wobei R³ ausgewählt ist aus der Gruppe bestehend aus Wasserstoff, Methyl, Ethyl, Propyl, Isopropyl, Butyl und Isobutyl; und
B unabhängig von A ausgewählt ist aus der Gruppe bestehend aus OH, SH, NHR⁴ und NH₂, wobei R⁴ ausgewählt ist aus der Gruppe bestehend aus Methyl, Ethyl und Propyl, wobei das Addukt **dadurch gekennzeichnet ist, dass** es keine freien isocyanatreaktiven Gruppen mehr aufweist.

2. Addukt gemäß Anspruch 1, wobei in Formel (II) A NR³ ist und R³ ausgewählt ist aus der Gruppe bestehend aus Wasserstoff, Methyl, Ethyl, Propyl, Isopropyl, Butyl und Isobutyl.

3. Addukt gemäß Anspruch 1, wobei in Formel (II) A Sauerstoff ist.

4. Addukt gemäß Anspruch 1, wobei R⁵ Butylen ist.

5. Addukt gemäß einem der Ansprüche 1 bis 4, wobei das Isocyanat ein monomeres oder oligomeres Isocyanat mit aliphatisch oder cycloaliphatisch gebundenen Isocyanatgruppen ist.

6. Addukt gemäß einem der Ansprüche 1 bis 5, umfassend wenigstens eine Verbindung mit wenigstens einer aliphatisch gebundenen Isocyanatgruppe und wenigstens eine weitere Verbindung mit wenigstens einer aromatisch gebundenen Isocyanatgruppe, wobei die erste Verbindung wenigstens 50 Gew.-% aller für die Herstellung des Addukts verwendeten Isocyanate ausmacht und die zweite Verbindung 5 Gew.-% bis 50 Gew.-% aller für die Herstellung des Addukts verwendeten Isocyanate ausmacht.

7. Verwendung eines Addukts gemäß einem der Ansprüche 1 bis 6 als Katalysator zur Vernetzung von wenigstens zwei Isocyanatgruppen ausgewählt aus der Gruppe bestehend aus aliphatisch, cycloaliphatisch, araliphatisch und aromatisch gebundenen Isocyanatgruppen.

8. Verwendung gemäß Anspruch 7, wobei die Verwendung zu einem hochvernetzten Polymer führt.

9. Polymerisierbare Zusammensetzung, enthaltend
a) wenigstens ein Polyisocyanat mit Isocyanatgruppen ausgewählt aus der Gruppe bestehend aus aliphatisch, cycloaliphatisch, araliphatisch und aromatisch gebundenen Isocyanatgruppen; und
b) wenigstens ein Addukt gemäß einem der Ansprüche 1 bis 6;
wobei das molare Verhältnis von Isocyanatgruppen zu isocyanatreaktiven Gruppen in der polymerisierbaren Zusammensetzung wenigstens 2:1 beträgt.

10. Polymerisierbare Zusammensetzung gemäß Anspruch 9, wobei die Polyisocyanatkomponente der polymerisierbaren Zusammensetzung zu wenigstens 80 Gew.-% aus wenigstens einem Polyisocyanat ausgewählt aus der Gruppe bestehend aus monomerem HDI, oligomerem HDI, monomerem PDI, oligomerem PDI, monomerem H12MDI, oligomerem H12MDI,monomerem IPDI und oligomerem IPDI besteht.

11. Kit, enthaltend
a) wenigstens ein Polyisocyanat mit Isocyanatgruppen ausgewählt aus der Gruppe bestehend aus aliphatisch, cycloaliphatisch, araliphatisch und aromatisch gebundenen Isocyanatgruppen und
b) wenigstens ein Addukt gemäß einem der Ansprüche 1 bis 6.

12. Verwendung der polymerisierbaren Zusammensetzung gemäß Anspruch 9 oder 10 oder des Kits gemäß Anspruch 11 zur Herstellung eines Polymers.

13. Verfahren zur Herstellung eines Polymers, enthaltend die Schritte
a) Vermischen wenigstens eines Polyisocyanats mit Isocyanatgruppen ausgewählt aus der Gruppe bestehend aus aliphatisch, cycloaliphatisch, araliphatisch und aromatisch gebundenen Isocyanatgruppen mit einem Addukt gemäß einem der Ansprüche 1 bis 6; und
b) Aushärten der in Verfahrensschritt a) erhaltenen polymerisierbaren Zusammensetzung durch Erhöhung der Temperatur auf wenigstens 50 °C,
wobei zu Beginn des Verfahrensschritts b) das Verhältnis von Isocyanatgruppen zu isocyanatreaktiven Gruppen in der polymerisierbaren Zusammensetzung wenigstens 2:1 beträgt.

14. Verfahren nach Anspruch 13, wobei zwischen dem Ende des Verfahrensschritts a) und dem Beginn des Verfahrensschritts b) ein Zeitraum von wenigstens 30 Minuten liegt.

15. Verfahren nach Anspruch 13 oder 14, **dadurch gekennzeichnet, dass** das in Verfahrensschritt a) erhaltene Reaktionsgemisch vor Durchführung des Verfahrensschritts b) mit einem organischen oder anorganischen Füllstoff vermischt wird.

16. Verfahren nach Anspruch 15, **dadurch gekennzeichnet, dass** der organische oder anorganische Füllstoff aus Fasern mit einer Mindestlänge von 50 m besteht und die Aushärtung in Verfahrensschritt b) in einem beheizten Werkzeug stattfindet, das dem mit dem Reaktionsgemisch benetzten Faserbündel ein Profil verleiht und dieses Profil durch die Aushärtung des Reaktionsgemisches stabilisiert.

17. Polymer, erhältlich durch das Verfahren gemäß Anspruch 13 oder 14, oder Verbundwerkstoff, erhältlich nach dem Verfahren gemäß Anspruch 15 oder 16.

## Revendications

1. Adduit d'un composé selon la formule (I) et d'un composé ayant au moins un groupe isocyanate 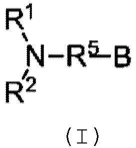
R¹ et R² étant indépendamment l'un de l'autre choisis dans le groupe constitué par hydrogène, méthyle, éthyle, propyle, isopropyle, butyle, isobutyle, C5-alkyle ramifié, C5-alkyle non ramifié, C6-alkyle ramifié, C6-alkyle non ramifié, C7-alkyle ramifié et C7-alkyle non ramifié ;
R⁵ étant choisi dans le groupe constitué par propylène, butylène, pentylène et un radical de formule (II), préférablement parmi butylène et le radical de formule (II) ; 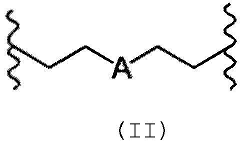
A dans la formule (II) étant choisi dans le groupe constitué par O, S et NR³, R³ étant choisi dans le groupe constitué par hydrogène, méthyle, éthyle, propyle, isopropyle, butyle et isobutyle ; et
B étant indépendamment de A choisi dans le groupe constitué par OH, SH NHR⁴ et NH₂, R⁴ étant choisi dans le groupe constitué par méthyle, éthyle et propyle, l'adduit étant **caractérisé en ce qu'**il ne comprend plus aucun groupe réactif envers un isocyanate libre.

2. Adduit selon la revendication 1, dans la formule (II) A étant NR³ et R³ étant choisi dans le groupe constitué par hydrogène, méthyle, éthyle, propyle, isopropyle, butyle et isobutyle.

3. Adduit selon la revendication 1, dans la formule (II) A étant oxygène.

4. Adduit selon la revendication 1, R⁵ étant butylène.

5. Adduit selon l'une quelconque des revendications 1 à 4, l'isocyanate étant un isocyanate monomérique ou oligomérique ayant des groupes isocyanate liés aliphatiquement ou cycloaliphatiquement.

6. Adduit selon l'une quelconque des revendications 1 à 5, comprenant au moins un composé ayant au moins un groupe isocyanate lié aliphatiquement et au moins un autre composé ayant au moins un groupe isocyanate lié aromatiquement, dans lequel le premier composé représente au moins 50 % en poids de tous les isocyanates utilisés pour la préparation de l'adduit et le deuxième composé représente 5 % en poids à 50 % en poids de tous les isocyanates utilisés pour la préparation de l'adduit.

7. Utilisation d'un adduit selon l'une quelconque des revendications 1 à 6 comme catalyseur pour la réticulation d'au moins deux groupes isocyanate choisis dans le groupe constitué par les groupes isocyanate liés aliphatiquement, cycloaliphatiquement, araliphatiquement et aromatiquement.

8. Utilisation selon la revendication 7, dans laquelle l'utilisation conduit à un polymère hautement réticulé.

9. Composition polymérisable contenant
a) au moins un polyisocyanate ayant des groupes isocyanate choisis dans le groupe constitué par les groupes isocyanate liés aliphatiquement, cycloaliphatiquement, araliphatiquement et aromatiquement ; et
b) au moins un adduit selon l'une quelconque des revendications 1 à 6 ;
dans laquelle le rapport molaire des groupes isocyanate sur les groupes réactifs envers un isocyanate dans la composition polymérisable est d'au moins 2:1.

10. Composition polymérisable selon la revendication 9, dans laquelle le composant polyisocyanate de la composition polymérisable est constitué par une proportion d'au moins 80 % en poids d'au moins un polyisocyanate choisi dans le groupe constitué par HDI monomérique, HDI oligomérique, PDI monomérique, PDI oligomérique, H12MDI monomérique, H12MDI oligomérique, IPDI monomérique et IPDI oligomérique.

11. Kit contenant
a) au moins un polyisocyanate ayant des groupes isocyanate choisis dans le groupe constitué par des groupes isocyanate liés aliphatiquement, cycloaliphatiquement, araliphatiquement et aromatiquement et
b) au moins un adduit selon l'une quelconque des revendications 1 à 6.

12. Utilisation de la composition polymérisable selon la revendication 9 ou 10 ou du kit selon la revendication 11 pour la production d'un polymère.

13. Procédé pour la production d'un polymère contenant les étapes de
a) mélange d'au moins un polyisocyanate ayant des groupes isocyanate choisis dans le groupe constitué par des groupes isocyanate liés aliphatiquement, cycloaliphatiquement, araliphatiquement et aromatiquement avec un adduit selon l'une quelconque des revendications 1 à 6 ; et
b) durcissement de la composition polymérisable obtenue à l'étape a) du procédé en élevant la température à au moins 50 °C,
dans lequel, au début de l'étape b) du procédé, le rapport des groupes isocyanate sur les groupes réactifs envers un isocyanate dans la composition polymérisable est d'au moins 2:1.

14. Procédé selon la revendication 13, dans lequel une période d'au moins 30 minutes s'écoule entre la fin de l'étape a) du procédé et le début de l'étape b) du procédé.

15. Procédé selon la revendication 13 ou 14, **caractérisé en ce que** le mélange réactionnel obtenu dans l'étape a) du procédé est mélangé avec une charge organique ou inorganique avant la réalisation de l'étape b) du procédé.

16. Procédé selon la revendication 15, **caractérisé en ce que** la charge organique ou inorganique est constituée de fibres d'une longueur minimale de 50 m et le durcissement dans l'étape b) du procédé est effectué dans un moule chauffé qui confère un profilé au faisceau de fibres mouillé avec le mélange réactionnel et stabilise ce profilé par durcissement du mélange réactionnel.

17. Polymère pouvant être obtenu par le procédé selon la revendication 13 ou 14 ou matériau composite pouvant être obtenu par le procédé selon la revendication 15 ou 16.
